(19) Europäisches Patentamt
European Patent Office
Office européen des brevets

(11) Publication number: **0 047 531**
**B1**

(12) # EUROPEAN PATENT SPECIFICATION

(45) Date of publication of patent specification: **12.06.85**

(21) Application number: **81107104.2**

(22) Date of filing: **09.09.81**

(51) Int. Cl.[4]: **C 07 D 405/06,**
C 07 D 405/04, A 61 K 31/415
// (C07D405/06, 233:64, 319:20)

(54) **2-(1,4-Benzodioxan-2-ylalkyl) imidazoles, their preparation and pharmaceutical compositions containing them.**

(30) Priority: **10.09.80 US 185832**

(43) Date of publication of application:
**17.03.82 Bulletin 82/11**

(45) Publication of the grant of the patent:
**12.06.85 Bulletin 85/24**

(84) Designated Contracting States:
**AT BE CH DE FR GB IT LI LU NL SE**

(56) References cited:
**US-A-4 039 676**

(73) Proprietor: **SYNTEX (U.S.A.) INC.**
**3401 Hillview Avenue**
**Palo Alto, California 94304 (US)**

(72) Inventor: **Kluge, Arthur F.**
**1683 Parkhills Avenue**
**Los Altos California 94022 (US)**
Inventor: **Strosberg, Arthur M.**
**120 Lucero Way**
**Portola Valley California 94025 (US)**
Inventor: **Whiting, Roger L.**
**114 Woodfield Avenue**
**Colinton Edinburgh EH14 2LX (GB)**
Inventor: **Christie, George A.**
**Boag's Mill 4 Bogsmill Road**
**Colinton Dell Edinburgh EH14 2LX (GB)**
Inventor: **Waterbury, David L.**
**10515 S. Foothill Blvd.**
**Cupertino California 95014 (US)**

(74) Representative: **Schmied-Kowarzik, Volker, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 047 531 B1**

**Description**

BACKGROUND OF THE INVENTION

*Field of the Invention*

The present invention is concerned with compounds, their preparation and compositions which exhibit selective $\alpha_2$ blockade in mammals, and which, therefore, are useful as medicaments relating to physiological functions affected by such blockade. Such activities include, for example, amelioration of depression, inhibition of platelet aggregation, palliation of diabetes and weight-loss stimulation. The compounds of the present invention are also effective in lowering intraoccular pressure, and are therefore useful in the treatment of diseases of the eye. In particular, compounds of benzodioxane substituted in the 2-position with substituents containing imidazolyl are thus useful.

*Prior Art*

A large number of compounds, in which the 1,4-benzodioxane system is substituted at the 2-position by a side chain containing nitrogen have been prepared, and shown to be active either in the central nervous system and/or the cardiovascular system. There appears to be no standard assay system for discriminating among the various types of effects of compounds on these target tissues; therefore the prior art is often non-specific as to the exact mode of action of the compounds tested. However, a variety of compounds having the general formula

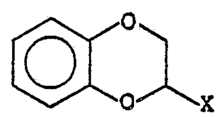

where X contains nitrogen in fairly close proximity to the ring are physiologically active. None is entirely satisfactory as an antidepressant. Those preparations closest in structure to the present invention are described in South African Patent 64/622, Canadian Patent 731,147, Belgian Patents 643,853 and 837,386, U.S. Patents 2,979,511, 3,360,529, 3,829,441, 3,944,549 and 3,959,283, British Patents 1,051,143 and 1,094,982, Japanese Patents 54/103,893, 55/015,456 and 55/015,455, and Dutch Patent Application 73,0718. No previous disclosure of these compounds as effective in controlling intraoccular pressure has been found.

SUMMARY OF THE INVENTION

Imidazole derivatives of the 1,4-benzodioxan-2-yl system having the general formula I

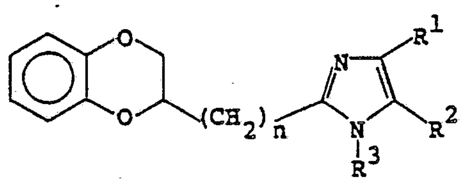

(I)

wherein $R^1$, $R^2$ and $R^3$ are independently selected from the group consisting of hydrogen and alkyl and n is an integer equal to 0, 1 or 2; and the pharmaceutically acceptable acid addition salts thereof, are novel.

Therefore, the present invention relates to compounds in the class described above, to the methods of preparation thereof, to pharmaceutical compositions containing such compounds.

DETAILED DESCRIPTION OF THE INVENTION

*Definitions:*

As used herein:

"alkyl" means a branched or unbranched saturated hydrocarbon chain containing 1—6, preferably 1—4, carbon atoms, such as methyl, ethyl, propyl, isopropyl, n-butyl, iso-butyl, tert-butyl or n-hexyl;

"pharmaceutically acceptable acid addition salts" refers to those salts which retain the biological effectiveness and properties of the free bases and which are not biologically or otherwise undesirable, formed with inorganic acids such as hydrochloric acid, hydrobromic acid, sulfuric acid, nitric acid or phosphoric acid, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid or salicylic acid.

*Preferred Compounds:*

A set of preferred compounds is that wherein n=1, and $R^1$, $R^2$ and $R^3$ are each independently selected from the group consisting of hydrogen, methyl, ethyl, isopropyl, n-propyl, and n-butyl; and the pharmaceutically acceptable acid addition salts thereof.

Especially preferred compounds are 1-methyl-2-(1,4-benzodioxan-2-ylmethyl)imidazole; 1-ethyl-2-(1,4-benzodioxan-2-ylmethyl)imidazole; and 1-n-propyl-2-(1,4-benzodioxan-2-ylmethyl)imidazole; and the pharmaceutically acceptable acid addition salts thereof.

Other preferred compounds are those wherein $R^1$, $R^2$ and $R^3$ are hydrogen and n = 0, 1 or 2, i.e.:

2-(1,4-benzodioxan-2-yl)imidazole;
2-(1,4-benzodioxan-2-ylmethyl)imidazole; or
2-[2-(1,4-dibenzodioxan-2-yl)ethyl]imidazole

and the pharmaceutically acceptable acid addition salts thereof.

*Preparation:*

Compounds of General Formula I are prepared by the reaction sequence:

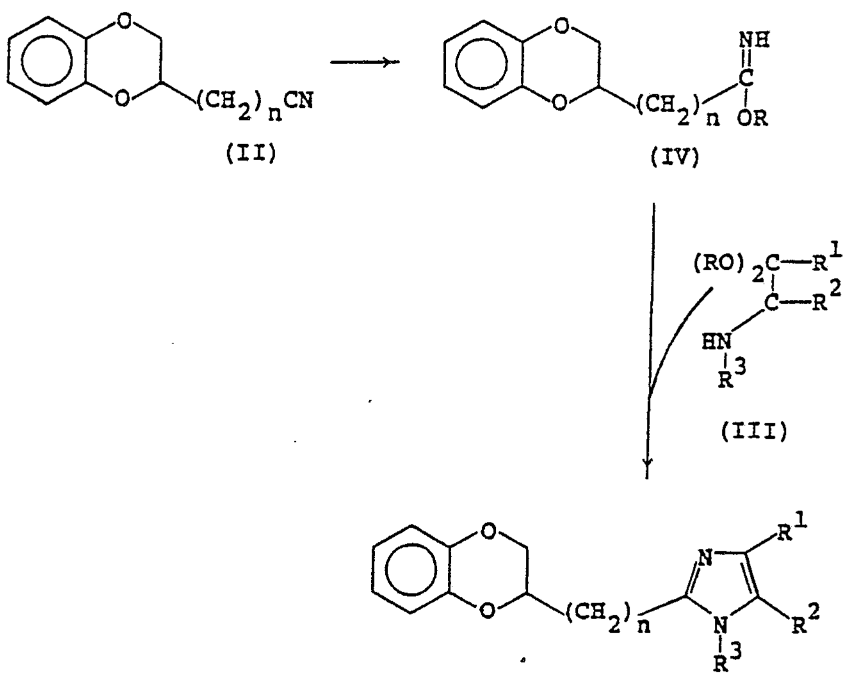

(II) (IV) (III)

(I)

wherein each R in (III) and (IV) is independently alkyl (1—6C) and the two R groups in (III) may or may not be cyclized.

In addition, subsequent to the above reaction sequence, compounds, of General Formula I wherein $R^3$ is hydrogen may be alkylated at the nitrogen of the imidazole ring to form other compounds of General Formula I wherein $R^3$ is alkyl. In those cases wherein $R^1$ and $R^2$ are not identical, such alkylation will of course, lead to mixtures of isomers which must then be separated by conventional means, such as, for example; fractional crystallization or chromatography. In such cases, subsequent alkylation of performed compounds of General Formula I would not, of course, be the method of choice; and such compounds would be better prepared by supplying the appropriate N-alkylated ketal.

The method for preparation of II is described by Augstein, et. al. in j. Med. Chem. 8, 446 (1965), and of III by Adachi and Sato in J. Org. Chem. 37, 221 (1972).

It is to be understood that isolation and purification of the compounds and intermediates described herein, whether in the body of the specification, or Examples, can be effected, if desired, by any suitable separation or purification procedure such as, for example, filtration, extraction, crystallization, column chromatography, thin-layer chromatography or thick-layer chromatography, or a combination of these procedures. Specific illustrations of suitable separation and isolation procedures can be had by reference to the Examples hereinbelow. However, other equivalent separation or isolation procedures could, of course, also be used.

The salt products are also isolated by conventional means. For example, the reaction mixtures may be evaporated to a dryness, and the salts can be further purified by conventional methods.

All of the compounds of General Formula I possess at least one chiral center, i.e., the number 2 carbon

of the dioxane ring at which substitution is made. Accordingly, the compounds of the present invention may be prepared in either optically active form or as racemic mixtures. Unless otherwise specified, the compounds described herein are all in the racemic form. However, the scope of the subject invention herein is not to be considered limited to the racemic forms, but to encompass the individual optical isomers of the compounds.

If desired, the compounds herein may be resolved into their optical antipodes by conventional resolution means; for example by separation (e.g. fractional crystallization) of the diastereomeric salts formed by the reaction of these compounds with optically active acids, at temperatures between 0°C and the reflux temperature of the solvent employed for fractional crystallization. Exemplary of such optically active acids are the optically active forms of camphor-10-sulfonic acid, 2-bromo-camphor-π-sulfonic acid, camphoric acid, menthoxyacetic acid, tartaric acid, malic acid, diacetyltartaric acid or pyrrolidine-5-carboxylic acid. The separated pure diastereomeric salts may then be cleaved by standard means to afford the respective optical isomers of the compounds of General Formula I.

In typical preparations, Compound(s) II is converted to IV by treatment with an excess of an alcohol under acidic conditions at low temperature, in the range of −10 to 10°C, and in the absence or presence of an aprotic organic solvent, such as, for example diethyl ether or tetrahydrofuran. A preferred temperature range is 1—5°C; preferred alcohols are methanol, ethanol and i-propanol; a preferred solvent is diethyl ether, and a preferred acid is anhydrous HCl.

The reaction mixture is allowed to stand at the aforementioned low temperature for several hours or days before being warmed to room temperature (15—30°C), and the crude product permitted to precipitate out.

The crude product is recovered and purified by conventional means; a particularly preferred isolation procedure is to recover the precipitated salt by filtration and purify it using thin layer chromatography. In this manner,

2-cyano-1,4-benzodioxane;
2-cyanomethyl-1,4-benzodioxane; and
2-(2-cyanoethyl)-1,4-benzodioxane

may be converted to the corresponding imidates or the acid addition salts thereof.

In the succeeding conversion of IV to I, treatment of IV with III leads to an intermediate of the General Formula V:

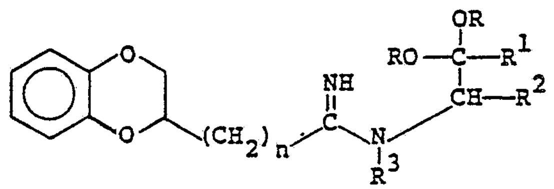

(V)

which may be if desired isolated in crude form for conversion by treatment with acid to I. However said isolation is pointless, and the conversion of IV to I is preferably effected in one step.

To effect this conversion approximately equimolar amounts of IV and III are dissolved in a polar organic solvent, such as methanol, ethanol or acetone, preferably ethanol. Reaction occurs at elevated temperatures 60—100°C, preferably at the reflux temperature of the solvent. Reaction time will vary with temperature, but may be effected within several hours. The product is recovered by conventional means, which may include solvent evaporation, filtration, removal of solvent under reduced pressure. Further purification may be effected, if desired.

Compound V is converted to I by treatment with aqueous acid. Suitable acids include hydrochloric, sulfuric, phosphoric, nitric, oxalic or acetic, at concentrations in the range of 1—6 N, preferably 2—4 N. The reaction occurs at temperature of 50—100°C; preferably 60—70°C. The crude product precipitates from the reaction mixture and is recovered as the salt and purified by conventional means, or may be converted to the free base by treatment with usual reagents such as sodium hydroxide, sodium carbonate or calcium hydroxide.

The subject compounds of the instant invention can thus, be isolated as free bases; however, because some compounds in base form are oils or gums, it is more convenient to isolate and characterize them as acid addition salts. These salts are prepared in the usual manner, i.e., by reaction of the base compound with a suitable inorganic or organic acid, described above. Salts formed with dibasic acids (e.g. oxalic acid) may contain one or two molecules of base per molecule of acid. If desired, the salts can be readily converted to the compounds in base form by treatment with alkali, such as potassium carbonate, sodium carbonate or sodium or potassium hydroxide.

Compounds of General Formula I wherein $R^3$ is hydrogen, may be converted to the corresponding N-alkylated compounds. To effect this conversion, the compound of General Formula I or its salt is dissolved in an aprotic organic solvent, such as, e.g. dimethoxyethane (DME), dimethyl formamide (DMF) or acetonitrile, preferably DMF, and an excess of alkali metal hydride is added, preferably NaH. The mixture is maintained at about 15 to 35°C, preferably 20—25°C for about 10 minutes to 2 hours, preferably 20—40

minutes. The appropriate alkyl halide (in an amount slightly in excess of I but less than the metal hydride) is added and the reaction carried out at about 10 minutes to 2 hours, preferably 20—40 minutes. The reaction mixture is then cooled and the product isolated by conventional means.

*Utility and Administration:*

The compounds of General Formula I and the pharmaceutically acceptable acid addition salts thereof exhibit CNS activity, and, in particular, have been shown to be $\alpha_2$ blockers in standard laboratory tests. Accordingly, these compounds and pharmaceutically acceptable compositions containing them are useful in prevention, reduction and inhibition of depression in mammals, including humans; and in regulation of other physiological phenomena related to $\alpha_2$ receptors.

These other physiological activities include, for example, inhibition of platelet aggregation, palliation of diabetes, and stimulation of weight loss. In addition, the compounds of the present invention have been shown to be effective in lowering intraoccular pressure, and hence are useful in treating diseases of the eye which elevate such pressure, the most common of which is glaucoma.

In applying the compounds of this invention to treatment of conditions which are regulated by the CNS, administration of the active compounds and salts described herein can be via any of the accepted modes of administration for agents which relieve depression or affect the central nervous system including oral, parenteral and otherwise systemic in the form of solid, semi-solid or liquid dosage forms, such as, for example, tablets, suppositories, pills, capsules, powders, liquids or suspensions, preferably in unit dosage forms suitable for single administration of precise dosages. The compositions will include a conventional pharmaceutical carrier or excipient and an active compound of General Formula I or the pharmaceutically acceptable salts thereof and, in addition, may include other medicinal agents, pharmaceutical agents, carriers, adjuvants, etc.

The amount of active compound administered will of course, be dependent on the subject being treated, the severity of the affliction, the manner of administration and the judgment of the prescribing physician. However, an effective dosage is in the range of 0.1—10 mg/kg/day, preferably 1—5 mg/kg/day. For an average 70 kg human, this would amount to 7—700 mg per day, or preferably 70—350 mg/day.

For solid compositions, conventional non-toxic solid carriers include, for example, pharmaceutical grades of mannitol, lactose, starch, magnesium stearate, sodium saccharin, talcum, cellulose, glucose, sucrose or magnesium carbonate may be used. The active compound as defined above may be formulated as suppositories using, for example, polyalkylene glycols, for example, propylene glycol, as the carrier. Liquid pharmaceutically administerable compositions can, for example, be prepared by dissolving, dispersing, etc. an active compound as defined above and optional pharmaceutical adjuvants in a carrier, such as, for example, water, saline, aqueous dextrose, glycerol or ethanol, to thereby form a solution or suspension. If desired, the pharmaceutical composition to be administered may also contain minor amounts of nontoxic auxiliary substances such as wetting or emulsifying agents or pH buffering agents, for example, sodium acetate, sorbitan monolaurate, triethanolamine sodium acetate, sorbitan monolaurate, triethanolamine oleate, etc. Actual methods of preparing such dosage forms are known, or will be apparent, to those skilled in this art; for example, see *Remington's Pharmaceutical Sciences,* Mack Publishing Company, Easton, Pennsylvania, 15th Edition, 1975. The composition or formulation to be administered will, in any event, contain a quantity of the active compound(s) in an amount effective to alleviate the symptoms of the subject being treated.

Dosage forms or compositions containing active ingredient (compound of General Formula I or its salts) in the range of 0.25 to 95% with the balance made up from non-toxic carrier may be prepared.

For oral administration, a pharmaceutically acceptable non-toxic composition is formed by the incorporation of any of the normally employed excipients, such as, for example pharmaceutical grades of mannitol, lactose, starch, magnesium stearate, sodium saccharin, talcum, cellulose, glucose, sucrose, magnesium or carbonate. Such compositions take the form of solutions, suspensions, tablets, pills, capsules, powders or sustained release formulations. Such compositions may contain 10%—95% active ingredient, preferably 25—70%.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol or ethanol. In addition, if desired, the pharmaceutical compositions to be administered may also contain minor amounts of non-toxic auxiliary substances such as wetting or emulsifying agents or pH buffering agents such as for example, sodium acetate, sorbitan monolaurate, triethanolamine oleate, etc.

A more recently devised approach for parenteral administration employs the implantation of a slow-release or sustained-release system, such that a constant level of dosage is maintained. See, e.g., U.S. Patent No. 3,710,795.

The percentage of active compound contained in such compositions is highly dependent on the specific nature thereof, as well as the activity of the compound and the needs of the subject. However, percentages of active ingredient of 0.25% to 10% are employable, (higher if the composition is a solid which will be subsequently diluted to the above percentages), preferably in the range of 1—2%.

For systemic administration via suppository, traditional binders and carriers include, e.g. polyalkalene

glycols or triglycerides. Such suppositories may be formed from mixtures containing active ingredient in the range of 0.5—10%; preferably 1—2%.

In applying the compounds of the invention to treatment of diseases or disorders of the eye which are associated with an abnormally high intraoccular pressure, administration is in the form of drops or a solution applied directly to the eye.

Such compositions are typically sterilized aqueous solutions (i.e. eyedrops) containing 0.001 to 10%, most preferably 0.005 to 1% of the active ingredient, along with suitable buffer, stabilizer, and preservative. The total concentration of solutes should be such that, if possible, the resulting solution is isotonic with the lacrimal fluid (though this is not absolutely necessary) and has an equivalent pH in the range of pH 6—8. Typical sterilants are phenyl mercuric acetate, thimerosal, chlorobutanol, and benzalkonium chloride. Typical buffer systems and salts are based on, for example, citrate, borate or phosphate; suitable stabilizers include glycerin and polysorbate 80. The aqueous solutions are formulated simply by dissolving the solutes in a suitable quantity of water, adjusting the pH to about 6.8—8.0, making a final volume adjustment with additional water, and sterilizing the preparation using methods known to those in the art.

The dosage level of the resulting composition will, of course, depend on the concentration of the drops, the condition of the subject and the individual magnitude of responses to treatment. However, typical dosage ranges might be about 2—10 drops of a 0.1% solution of active ingredient per day.

The following preparation and examples illustrate the invention.

PREPARATION I

Preparation of ethyl (1,4-benzodioxan-2-yl)acetimidate hydrochloride

17.5 g (0.10 mole) 2-cyanomethyl-1,4-benzodioxane, prepared as described by augstein, et al, J. Med. Chem. 8: 446 (1965), was dissolved in a mixture containing 7 g ethanol and 50 ml diethyl ether. 4.5 g (0.15 moles) of dry HCl was bubbled though the mixture, which was then capped. The mixture was allowed to stand at 5°C for 4 days, followed by 3 days at room temperature. The crude product imidate hydrochloride (IV) precipitated out and was harvested by filtration, and washed with 100 ml ether, followed by 3 × 100 ml portions of methylene chloride. The solid was then purified by thin layer chromatography using 10% methanol in chloroform as a developing solvent. The product has an $R_f$ value of 0.7—0.8; starting material moves farther in this solvent system, and none was present in the crude product. The yield of product was 15.3 g, as the hydrochloride, or 59% yield.

Example 1

Conversion of ethyl (1,4-benzodioxan-2-yl) acetimidate · HCl to 2-(1,4-benzodioxan-2-ylmethyl)imidazole · HCl

The imidate hydrochloride was further reacted as follows: 5.15 g (0.02 moles) of this material and 2.93 g (0.02 moles) of aminoacetaldehyde diacetal were placed in 70 ml ethanol and the mixture was refluxed overnight. The crude intermediate (V) was harvested by filtration, the solvent was removed, and trituration with ether gave 6.7 g of a brown oil. The oil was then stored with 100 ml 3N HCl at 60—70°C for 24 hours. The crude product (I) was harvested by filtration and solvent removed. The product was then recrystallized three times from isopropanol to give 1.8 g of white crystalline hydrochloride, m.p. 221—224°C(d), or a 36% yield from the imidiate hydrochloride (IV).

In a similar manner, substituting other starting materials of General Formula IV for ethyl(1,4-benzodioxan-2-yl)acetimidate, for example

ethyl (1,4-benzodioxan-2-yl)formimidate; and
ethyl 3-(1,4-benzodioxan-2-yl)propionimidate is productive of the corresponding products:
2-(1,4-benzodioxan-2-yl)imidazole; m.p. 225—227°C as the hydrochloride; and
2-[2-(1,4-benzodioxan-2-yl)ethyl]imidazole; m.p. 159—160°C as the hydrochloride.

Similarly, substituting other acetals or ketals of General Formula III for aminoacetylaldehyde diethyl acetal such as those wherein $R^1$, $R^2$ and $R^3$ are the same or different and are independently selected from the group consisting of hydrogen and lower alkyl (alkyl being defined as above) for example:

N-methylaminoacetaldehyde diethyl acetal;
2-aminopropionaldehyde diethyl acetal;
3-amino-2-propanone diethyl ketal;
2-(N-methylamino)-propionaldehyde diethyl acetal;
3-(N-methylamino)-2-propanone diethyl ketal;
3-amino-2-butanone diethyl ketal;
3-(N-methylamino)-2-butanone diethyl ketal;
N-ethylamino-acetaldehyde diethyl acetal;
N-propylamino-acetaldehyde diethyl acetal is productive of the products:
1-methyl-2-(1,4-benzodioxan-2-ylmethyl)imidazole; m.p. 158—160°C as the hydrochloride
5-methyl-2-(1,4-benzodioxan-2-ylmethyl)imidazole;
4-methyl-2-(1,4-benzodioxan-2-ylmethyl)imidazole;
1,5-dimethyl-2-(1,4-benzodioxan-2-ylmethyl)imidazole;

1,4-dimethyl-2-(1,4-benzodioxan-2-ylmethyl)imidazole;
4,5-dimethyl-2-(1,4-benzodioxan-2-ylmethyl)imidazole;
1,4,5-trimethyl-2-(1,4-benzodioxan-2-ylmethyl)imidazole;
1-ethyl-2-(1,4-benzodioxan-2-ylmethyl)imidazole; m.p. 174—175°C as the hydrochloride; and
1-propyl-2-(1,4-benzodioxan-2-ylmethyl)imidazole; m.p. 123—124°C as the hydrochloride.

Example 2

1-ethyl-2-(1,4-benzodioxan-2-yl)imidazole hydrochloride

A. The hydrochloride salt of 2-(1,4-benzodioxan-2-yl)imidazole (1.25 g, 5 mmol) was dissolved in dimethylformamide (20 ml) and 50% sodium hydride (0.58 g, 12 mmol) was added. The mixture was stirred at room temperature for 30 minutes and ethyl iodide (1.09 g, 7 mmol) was then added. The solution was heated to 60°C for 30 minutes, cooled, added to water (100 ml), and extracted with ethyl acetate. The ethyl acetate extract was washed with water, dried, and evaporated to a solid residue. The solid was dissolved in methanolic hydrogen chloride and ether was added to induce crystallization. The salt was recrystallized from isopropanol-ether to afford 1-ethyl-2-(1,4-benzodioxan-2-yl)benzimidazole hydrochloride, 0.54 g, m.p. 174—175°.

B. Similarly, substituting for ethyliodide, in part A of this example,
methyl iodide
n-propyl iodide
t-butyl iodide
the corresponding 1-methyl, 1-n-propyl, and 1-t-butyl-2-(1,4-benzodioxan-2-yl)imidazoles are obtained.

Example 3

Conversion of 2-(1,4-benzodiozan-2-ylmethyl)imidazole to its hydrochloride

Excess 3% hydrogen chloride in methanol is added to a solution of 1.0 g. 2-(1,4-benzodioxan-2-ylmethyl)imidazole in 20 ml methanol. Diethyl ether is added until precipitation is complete. The product is filtered, washed with ether, air dried and recrystallized from methanol/acetone to yield 2-(1,4-benzodioxan-2-ylmethyl)imidazole hydrochloride, m.p.221—224°C(d).

In a similar manner, all compounds of General Formula I in free base form may be converted to the acid addition salts by treatment with the appropriate acid, for example, hydrochloric acid, hydrobromic acid, sulfuric acid, nitric acid, phosphoric acid, acetic acid, propionic acid, glycolic acid, pyruvic acid, oxalic acid, malonic acid, succinic acid, malic acid, maleic acid, fumaric acid, tartaric acid, citric acid, benzoic acid, cinnamic acid, mandelic acid, methanesulfonic acid, ethanesulfonic acid or p-toluenesulfonic acid.

Example 4

Conversion of a salt of 2-(1,4-benzodioxan-2-ylmethyl)imidazole to free base

1-Ethyl-2-(1,4-benzodioxan-2-ylmethyl)imidazole hydrochloride (1.0 g) suspended in 50 ml of ether is stirred with excess dilute aqueous potassium carbonate solution until the salt is completely dissolved. The organic layer is then separated, washed twice with water, dried over magnesium sulfate and evaporated to yield 1-ethyl-2-(1,2-benzodioxan-2-ylmethyl)imidazole, m.p. 77—78°C.

In a similar manner the acid addition salts of all compounds of General Formula I may be converted to the corresponding compounds in free base form.

Example 5

Direct interchange of acid addition salts of 1-ethyl-2-(1,4-benzodioxan-2-ylmethyl)imidazole

1-Ethyl-2-(1,4-benzodioxan-2-ylmethyl)imidazolium acetate (1.0 g) is dissolved in 50 ml 50% aqueous sulfuric acid, and the solution evaporated to dryness. The product is suspended in ethanol and filtered, air dried and recrystallized from methanol/acetone to yield di(1-ethyl-2-(1,4-benzodioxan-2-ylmethyl)-imidazolium) sulfate, m.p. 115—116°C.

In a similar manner, but substituting for sulfuric acid, phosphoric or nitric acid, one obtains respectively 1-ethyl-2-(1,4-benzodioxan-2-ylmethyl)imidazolium dihydrogen phosphate, m.p. 180—182°C, and 1-ethyl-2-(1,4-benzodioxan-2-ylmethyl)imidazolium nitrate, m.p. 98—99°C.

In Examples 6 through 9, the active ingredient is 2-(1,4-benzodioxan-2-ylmethyl)imidazole hydrochloride. Other compounds of General Formula I and the pharmaceutically acceptable salts thereof may be substituted therein.

Example 6

Compositions for Oral Administration

| The composition contains: | % wt./wt. |
|---|---|
| Active ingredient | 95% |
| Lactose | 5% |

The two ingredients are milled, mixed and dispensed into capsules containing 100 mg each; one capsule would approximate a total daily dosage.

Example 7

Composition for Oral Administration

| The composition contains: | % wt./wt. |
|---|---|
| Active ingredient | 56.8% |
| Magnesium stearate | 0.9% |
| Starch | 8.6% |
| Lactose | 32.9% |
| PVp (polyvinylpyrrolidine) | 0.9% |

The above ingredients are combined and granulated using methanol as solvent. The formulation is then dried and formed into tablets (containing 200 mg of active compound) with an appropriate tableting machine.

Example 8

Parenteral Formulation (IV)

| The composition contains: | % wt./wt. |
|---|---|
| Active ingredient | 0.25 g |
| Propylene glycol | 20 g |
| Polyethylene glycol 400 | 20 g |
| Polysorbate 80 | 1 g |
| 0.9% Saline solution qs ad | 100 ml |

The active ingredient is dissolved in propylene glycol, polyethylene glycol 400 and polysorbate 80. A sufficient quantity of 0.9% saline solution is then added with stirring to provide 100 ml. of the I.V. solution which is filtered through a 0.2 micron membrane filter and packaged under sterile conditions.

Example 9

Suppository Formulation

| The composition contains: | % wt./wt. |
|---|---|
| Active ingredient | 1.0% |
| Polyethylene glycol 1000 | 74.5% |
| Polyethylene glycol 4000 | 24.5% |

The ingredients are melted together and mixed on a steam bath, and poured into molds containing 2.5 g total weight.

Example 10
Composition for Topical Administration to the Eye

| The composition contains: | % wt/vol |
|---|---|
| Active ingredient | 0.10 |
| Benzalkonium chloride | 0.02 |
| EDTA | 0.01 |
| Phenylethanol | 0.25 |
| Boric acid | 1.62 |
| NaOH | to adjust pH |
| water qs and | 100 ml |

The first four ingredients are dissolved in less than the required total volume of water, and the pH adjusted to 7.4. The volume is then brought to 100 ml with additional water.

Example 11
Assay for pre- and post-synaptic α-adrenoceptor blockade

*Protocol:*

Contralateral, prostatic and epididymal portions of the rat isolated vas deferens were suspended in separate organ baths containing oxygenated Krebs — bicarbonate solution at 37°C. The test compound was added to the Krebs — bicarbonate solution bathing the epididymal and prostatic portions of vas deferens. The contralateral portions served as control tissues. All tissues were then allowed to equilibrate with the bathing solution for 30 minutes.

Pre-synaptic α-adrenoceptor blockade was determined using the prostatic portions of vas deferens. Following the equilibration period, dose-response curves for the inhibitory effect of xylazine on the contractile response of the vas deferens to single pulse nerve stimulation were obtained.

Post-synaptic α-adrenoceptor blockade was determined using the epididymal portions of rat vas deferens. Following the equilibration period, dose-response curves for the contractile effects of phenylephrine on the vas deferens were obtained.

*Results:*

The antagonistic potency of the test compounds at α-adrenoceptors was expressed in terms of their "$pA_2$" values for each receptor. (The numbers in parentheses represent the number of determinations.) These values are the negative logarithms of the ratio of the doses of agonist causing 50% of the maximal response in the presence and absence of the test compound, according to the method of Arunlakshana and Shild, Brit. J. Pharmacol.; 14: 48—58 (1959). The selectivity ratio is the antilogarithm of the difference between $pA_2(\alpha_2)$ and $pA_2(\alpha_1)$.

The following results were obtained:

| | $pA_2(\alpha_2)$ | $pA_2(\alpha_1)$ | $\alpha_2/\alpha_1$ |
|---|---|---|---|
| 2-(1,4-benzodioxan-2-ylmethyl)imidazole | 4.22(7) | 6.10(8) | 75.9 |
| 1-methyl-2-(1,4-benzodioxan-2-ylmethyl)imidazole | 6.20 | 4.30 | 75 |
| 1-ethyl-2-(1,4-benzodioxan-2-ylmethyl)imidazole | | | |
| (lot No. 1) | 6.79(4) | 4.00(1) | 616.0 |
| (lot No. 2) | 6.41(4) | 4.00(4) | 257.0 |
| 1-propyl-2-(1,4-benzodioxan-2-ylmethyl)imidazole | 6.57(4) | 4.20(3) | 234.0 |
| 2-(2-1,4-benzodioxan-2-ylethyl)imidazole | 4.21(3) | 6.21(3) | 100 |

### Example 12

### $LD_{50}$ for 1-ethyl-2-(1,4-benzodioxan-2-ylmethyl)imidazole

A range of quantities of 1-ethyl-2-(1,4-benzodioxan-2-ylmethyl)imidazole was injected intraperitonially into mice, and the behavior of the subjects observed. It was found that one-half of the animals died at a dose level of 200 mg/kg body weight. For 1-n-propyl-2-(1,4-benzodioxan-2-ylmethyl)imidazole the $LD_{50}$ was, also, 200 mg/kg. For 2-(1,4-benzodioxan-2-ylmethyl)imidazole the $LD_{50}$ was 70 mg/kg. All other compounds exhibit similar toxicity.

### Example 13

### Determination of Platelet Aggregation Inhibition Protocol

Blood platelets are collected in the standard manner, and incubated in an Aggregation Module Incubator-Cuvette in the presence of either the inhibitor to be tested, or without said inhibitor as a control. The aggregation of the platelets is observed after the addition of an inducer, and the samples are evaluated for the presence of a lag period and the slope of the aggregation curve, as well as the maximum height of the aggregation curve in comparison to the control. $IC_{50}$ values i.e. the concentration of inhibitor required for 50% inhibition, can be calculated from the inflection point on the appopriate dose response curve. Results: The following data were obtained for 1-ethyl-2-(1,4-benzodioxane-2-ylmethyl)imidazole as the hydrochloride salt.

| Inducer | $IC_{50}$ From Maximum Height (ΔT) | From Slope (Rate) |
|---|---|---|
| Arachidonic acid (2.5 × 10—4M) | 1.9 × 10—4M | 3.1 × 10—4M |
| ADP (5 × 10—6M) | 10—3M | 20% at 10—3M |
| Collagen 30λ) | 27% inhibition at 5 × 10—4M | 4.5 × 10—4M |
| Epinephrine (5 × 10—6M) | 5% inhibition at 5 × 10—4M | 99% inhibition 5 × 10—5M |

### Example 14

### Determination of Effect on Intraoccular Pressure

*Protocol:*

The compound to be tested is dissolved in saline, and applied topically to the eye. The intraoccular pressure is measured immediately before application, and at specified time intervals thereafter, by means of a probe which measures the force necessary to flatten a small area of corneal surface, according to the method described by Moses, R. A., Tr. Am. Acad. Opth. and Otol., Jan-Feb 1962: 88—95. Results are expressed in torr (mm Hg).

*Results:*

A 0.1% solution of 2-(1,4-benzodioxan-2-ylmethyl)-imidazole was applied to the right and left eyes of New Zealand white rabbits and the following pressure readings were obtained.

| | | Average Pressure | | | |
|---|---|---|---|---|---|
| | No. Rabbits | Initial | 2 hr | 4 hr | 6 hr |
| Expt 1 | 6 | 18.3 | 15.3 | — | — |
| Expt 2 | 6 | 18.0 | 16.1 | 16.5 | 18.0 |

**Claims**

1. A compound of the general Formula I

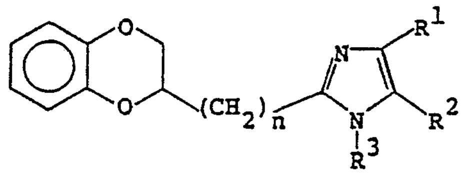

(I)

wherein $R^1$, $R^2$ and $R^3$ are independently selected from the group consisting of hydrogen and alkyl (1—6C), and wherein n is either 0, 1, or 2, and the pharmaceutically acceptable acid addition salts thereof.

2. The compound of Claim 1 wherein n = 1, and $R^1$, $R^2$ and $R^3$ are each independently selected from the group consisting of hydrogen, methyl, ethyl, isopropyl, n-propyl, and n-butyl, and the pharmaceutically acceptable acid addition salts thereof.

3. The compound of Claim 1 wherein n = 1, and $R^1$, $R^2$ and $R^3$ are hydrogen, i.e. 2-(1,4-benzodioxan-2-ylmethyl)imidazole, and the pharmaceutically acceptable acid addition salts thereof.

4. The compound of Claim 1 wherein n = 0 and $R^1$, $R^2$ and $R^3$ are hydrogen, i.e., 2-(1,4-benzodioxan-2-yl)imidazole, and the pharmaceutically acceptable acid addition salts thereof.

5. The compound of Claim 1 wherein n = 2 and $R^1$, $R^2$ and $R^3$ are hydrogen, i.e., 2-[(1,4-benzodioxan-2-yl)ethyl]imidazole, and the pharmaceutically acceptable acid addition salts thereof.

6. The compound of Claim 2 wherein $R^3$ is methyl, $R^1$ and $R^2$ are hydrogen and n = 1, i.e. 1-methyl-2-(1,4-benzodioxan-2-yl-methyl)imidazole and the pharmaceutically acceptable acid addition salts thereof.

7. The compound of Claim 2 wherein $R^3$ is ethyl, $R^1$ and $R^2$ are hydrogen and n = 1, i.e. 1-ethyl-2-(1,4-benzodioxan-2-ylmethyl)imidazole and the pharmaceutically acceptable acid addition salts thereof.

8. The compound of Claim 2 wherein $R^3$ is n-propyl, $R^1$ and $R^2$ are hydrogen and n = 1, i.e. 1-n-propyl-2-(1,4-benzodioxan-2-ylmethyl)imidazole.

9. A pharmaceutical composition comprising a therapeutically effective amount of a compound of the general Formula I

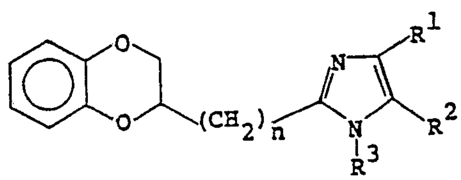

(I)

wherein $R^1$, $R^2$ and $R^3$ are independently selected from the group consisting of hydrogen and alkyl (1—6C), and wherein n is either 0, 1, or 2, or a pharmaceutically acceptable salt thereof.

10. A process for the production of a compound of the general formula I

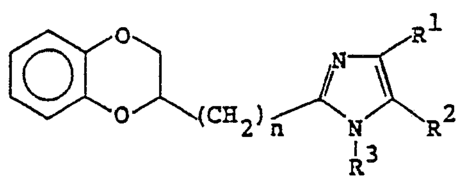

(I)

wherein $R^1$, $R^2$ and $R^3$ are independently selected from the group consisting of hydrogen and alkyl (1—6C), and wherein n is either 0, 1, or 2, and a pharmaceutically acceptable acid addition salt thereof which comprises one or more of the following steps

(a) converting an imidate of the general Formula IV

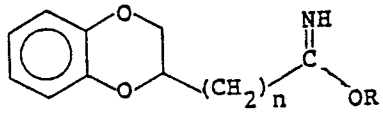

(IV)

wherein R is alkyl (1—6C) and n is 0, 1 or 2 to the corresponding compound of general Formula I by treatment with a ketal of the general Formula III

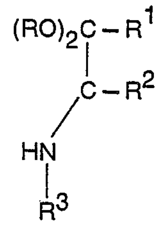

(III)

wherein R is alkyl (1—6C) and $R^1$, $R^2$ and $R^3$ are as herein defined; followed by treating with acid; or

(b) converting a ketal of the general Formula V

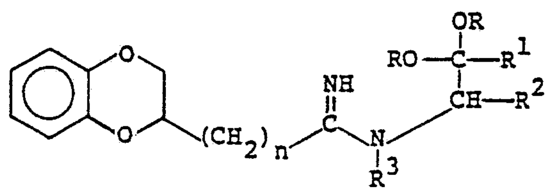

(V)

wherein $R^1$, $R^2$, $R^3$ and R are as herein defined, to the corresponding compound of general Formula I by treating with acid; or

(c) converting a compound of the general Formula I wherein $R^3$ is hydrogen to a compound of general Formula I wherein $R^3$ is alkyl by treating with the corresponding halide of $R^3$ in the presence of strong base; and optionally;

(d) converting a pharmaceutically acceptable acid addition salt to the free base; or

(e) converting the free base to the pharmaceutically acceptable acid addition salt; or

(f) converting a salt of the subject compound to another salt by a salt interchange.

**Patentansprüche**

1. Eine Verbindung der allgemeinen Formel I

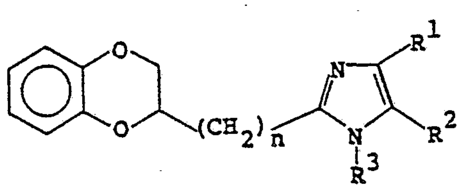

(I)

in welcher $R^1$, $R^2$ und $R^3$ unabhängig ausgewählt sind aus der aus Wasserstoff und $C_1$—$C_6$ Alkyl bestehenden Gruppe und n entweder 0, 1 oder 2, ist, und die pharmazeutisch annehmbaren Säureadditionssalze derselben.

2. Verbindung nach Anspruch 1, in welcher n = 1 ist und $R^1$, $R^2$ und $R^3$ jeweils unabhängig ausgewählt sind aus der aus Wasserstoff, Methyl, Ethyl, Isopropyl, n-Propyl und n-Butyl bestehenden Gruppe, und die pharmazeutisch annehmbaren Säureadditionssalze derselben.

3. Verbindung nach Anspruch 1, in welcher n = 1 ist und $R^1$, $R^2$ und $R^3$ Wasserstoff sind, d.h. das 2-(1,4-Benzodioxan-2-yl-methyl)-imidazol, und die pharmazeutisch annehmbaren Säureadditionssalze desselben.

4. Verbindung nach Anspruch 1, in welcher n = 0 ist und $R^1$, $R^2$ und $R^3$ Wasserstoff sind, d.h. das 2-(1,4-Benzodioxan-2-yl)-imidazol, und die pharmazeutisch annehmbaren Säureadditionssalze desselben.

5. Verbindung nach Anspruch 1, in welcher n = 2 ist und $R^1$, $R^2$ und $R^3$ Wasserstoff sind, d.h. das 2-[(1,4-Benzodioxan-2-yl)ethyl]-imidazol, und die pharmazeutisch annehmbares Säureadditionssalze desselben.

6. Verbindung nach Anspruch 2, in welcher $R^3$ Methyl ist, $R^1$ und $R^2$ Wasserstoff sind und n = 1 ist, d.h. das 1-Methyl-2-(1,4-benzodioxan-2-ylmethyl)-imidazol, und die pharmazeutisch annehmbaren Säureadditionssalze desselben.

7. Verbindung nach Anspruch 2, in welcher $R^3$ Ethyl ist, $R^1$ und $R^2$ Wasserstoff sind und n = 1 ist, d.h. das 1-Ethyl-2-(1,4-benzodioxan-2-ylmethyl)-imidazol, und die pharmazeutisch annehmbaren Säureadditionssalze desselben.

8. Verbindung nach Anspruch 2, in welcher $R^3$ n-Propyl ist $R^1$ und $R^2$ Wasserstoff sind und n = 1 ist, d.h. das 1-n-Propyl-2-(1,4-benzodioxan-2-ylmethyl)-imidazol.

9. Pharmazeutische Zusammensetzung, enthaltend eine therapeutisch wirksame Menge einer Verbindung der allgemeinen Formel I

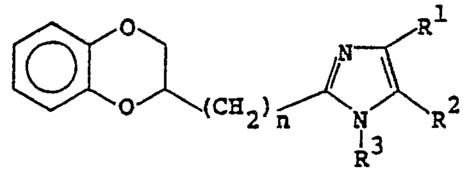

(I)

in welcher $R^1$, $R^2$ und $R^3$ unabhängig ausgewählt sind aus der aus Wasserstoff und $C_1$—$C_6$ Alkyl bestehenden Gruppe und n entweder 0, 1 oder 2 ist, oder eines pharmazzeutisch annehmbaren Salzes derselben.

10. Verfahren zur Herstellung einer Verbindung der allgemeinen Formel I

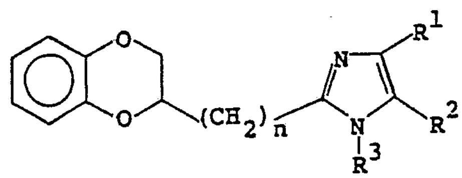

(I)

in welcher $R^1$, $R^2$ und $R^3$ unabhängig ausgewählt sind aus der aus Wasserstoff und $C_1$—$C_6$ Alkyl bestehenden Gruppe und n entweder 0, 1 oder 2 ist, oder eines pharmazeutische annehmbaren Säureadditionssalzes derselben, gekennziechnet durch eine oder mehrere der folgenden Stufen

(a) Umwandlung eines Imidates der allgemeinen Formel IV

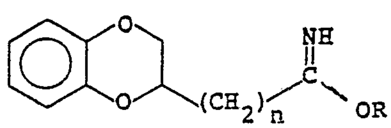

(IV)

in welcher R $C_1$—$C_6$ Alkyl ist und n 0, 1 oder 2 ist, durch Behandlung mit einem Ketal der allgemeinen Formel III

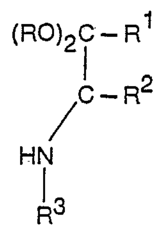

(III)

in welcher R $C_1$—$C_6$ Alkyl ist und $R^1$, $R^2$ und $R^3$ wie oben definiert sind, und anschließende Behandlung mit einer Säure in die entsprechende Verbindung der allgemeinen Formel I; oder

(b) Umwandlung eines Ketals der allgemeinen Formel V

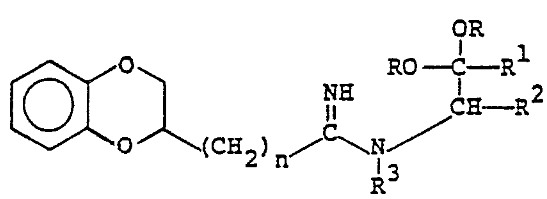

(V)

in welcher $R^1$, $R^2$ und $R^3$ wie oben definert sind, durch Behandlung mit einer Säure in die entsprechende Verbindung der allgemeinen Formel I; oder

(c) Umwandlung einer Verbindung der allgemeinen Formel I, in welcher $R^3$ Wasserstoff ist, in eine Verbindung der allgemeinen Formel I, in welcher $R^3$ Alkyl ist, durch Behandlung mit dem entsprechenden Halogenid von $R^3$ in Anwesenheit einer starken Base; und wahlweise

(d) Umwandlung eines pharmazeutisch annehmbaren Säureadditionssalzes in die freie Base; oder

(e) Umwandlung der freien Base in das pharmazeutisch annehmbare Säureadditionssalz; oder

(f) Umwandlung eines Salzes der betreffenden Verbindung in ein anderes Salz durch Salzaustausch.

**Revendications**

1. Composé de formule générale I

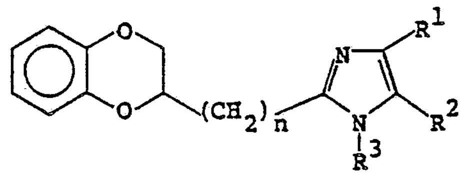

(I)

dans laquelle $R^1$, $R^2$ et $R^3$ sont choisis, indépendamment, dans le groupe comprenant l'hydrogène et un groupe alkyle en $C_1$ à $C_6$, et *n* a la valeur 0, 1 ou 2, et ses sels d'addition d'acides pharmaceutiquement acceptables.

2. Composé suivant la revendication 1, dans lequel *n* est égal à 1 et $R^1$, $R^2$ et $R^3$ sont choisis chacun, indépendamment, dans le groupe comprenant l'hydrogène, et les restes méthyle, éthyle, isopropyle, n-propyle et n-butyle, et les sels d'addition d'acides pharmaceutiquement acceptables de ce composé.

3. Composé suivant la revendication 1, dans lequel *n* est égal à 1 et $R^1$, $R^2$ et $R^3$ représentent l'hydrogène , c'est-à-dire le 2-(1,4-benzodioxanne-2-ylméthyl)imidazole, et ses sels d'addition d'acides pharmaceutiquement acceptables.

4. Composé suivant la revendication 1, dans lequel *n* est égal à 0 et $R^1$, $R^2$ et $R^3$ représentent l'hydrogène, c'est-à-dire le 2-(1,4-benzodioxanne-2-yl)imidazole, et ses sels d'addition d'acides pharmaceutiquement acceptables.

5. Composé suivant la revendication 1, dans lequel *n* est égal à 2 et $R^1$, $R^2$ et $R^3$ représent l'hydrogène, c'est-à-dire le 2-[(1,4-benzodioxanne-2-yl)éthyl]imidazole, et ses sels d'addition d'acides pharmaceutiquement acceptables.

6. Composé suivant la revendication 2, dans lequel $R^3$ est le groupe méthyle, $R^1$ et $R^2$ représentent l'hydrogène et *n* est égal à 1, c'est-à-dire le 1-méthyl-2-(1,4-benzodioxanne-2-ylméthyl)imidazole, et ses sels d'addition d'acides pharmaceutiquement acceptables.

7. Composé suivant la revendication 2, dans lequel $R^3$ est le groupe éthyle, $R^1$ et $R^2$ représentent l'hydrogène et *n* est égal à 1, c'est-à-dire le 1-éthyl-2-(1,4-benzodioxanne-2-ylméthyl)imidazole, et ses sels d'addition d'acides pharmaceutiquement acceptables.

8. Composé suivant la revendication 2, dans lequel $R^3$ est le group n-propyle, $R^1$ et $R^2$ représentent l'hydrogène et *n* est égal à 1, c'est-à-dire le 1-n-propyl-2-(1,4-benzodioxanne-2-ylméthyl)imidazole.

9. Composition pharmaceutique comprenant une quantité thérapeutique efficace d'un composé de formule générale I

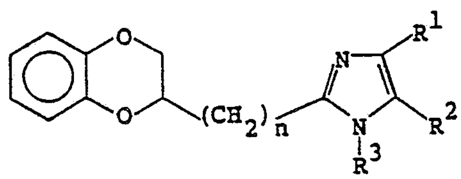

(I)

dans laquelle $R^1$, $R^2$ et $R^3$ sont choisis, indépendamment, dans le groupe comprenant l'hydrogène et des restes alkyle en $C_1$ à $C_6$, et *n* a la valeur 0, 1 ou 2, ou un sel pharmaceutiquement acceptable de ce composé.

10. Procédé de production d'un composé de formule générale I

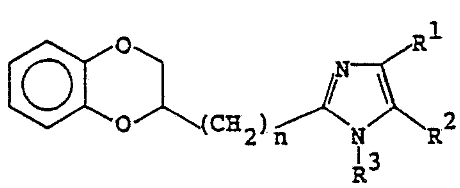

(I)

dans laquelle $R^1$, $R^2$ et $R^3$ sont choisis, indépendamment, dans le group comprenant l'hydrogène et des restes alkyle en $C_1$ à $C_6$, et *n* a la valeur 0, 1 ou 2, et d'un sel d'addition d'acide pharmaceutiquement acceptable de ce composé, qui comprend une ou plusieurs des étapes suivantes

(a) transformation d'un imidate de formule générale IV

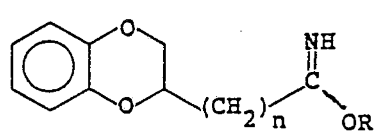

(IV)

dans laquelle R est un groupe alkyle en $C_1$ à $C_6$ et *n* a la valeur 0, 1 ou 2 en le composé correspondant de formule générale I par traitement avec un cétal de formule générale III

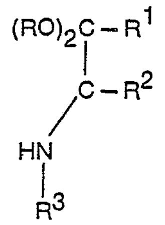

(III)

dans laquelle R est un groupe alkyle en $C_1$ à $C_6$ et $R^1$, $R^2$ et $R^3$ ont les définitions données ici; suivie d'un traitement avec un acide; ou

(b) transformation d'un cétal de formule générale V

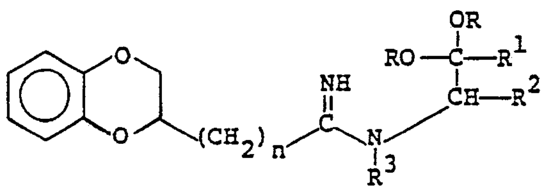

(V)

dans laquelle $R^1$, $R^2$, $R^3$ et R ont les définitions données ici, en le composé correspondant de formule générale I par traitement avec un acide; ou

(c) transformation d'un composé de formule générale I dans laquelle $R^3$ est l'hydrogène en un composé de formule générale I dans laquelle $R^3$ est un groupe alkyle par traitement avec l'halogénure correspondant de $R^3$ en présence d'une base forte; et à titre facultatif;

(d) transformation d'un sel d'addition d'acide pharmaceutiquement acceptable en la base libre; ou

(e) transformation de la base libre en le sel d'addition d'acide pharmaceutiquement acceptable; ou

(f) transformation d'un sel du composé en question en un autre sel par échange mutuel entre sels.